Europäisches Patentamt

European Patent Office

Office européen des brevets

(11) Publication number: **0 305 107**
**A2**

## (12) EUROPEAN PATENT APPLICATION

(21) Application number: **88307618.4**

(51) Int. Cl.⁴: **G01B 11/24**

(22) Date of filing: **17.08.88**

(30) Priority: **24.08.87 GB 8719951**

(43) Date of publication of application:
**01.03.89 Bulletin 89/09**

(84) Designated Contracting States:
**AT BE CH DE ES FR GB GR IT LI LU NL SE**

(71) Applicant: **L.B.P. Partnership**
**1706 Washington Avenue**
**St. Louis Missouri 63103(US)**

(72) Inventor: **Cruickshank, John Smith 39**
**Meadowview Drive**
**Craigdarroch Park**
**Inchture Perthshire Scotland(GB)**

(74) Representative: **Molyneaux, Martyn William et al**
**c/o Ladas & Parry Isartorplatz 5**
**D-8000 Munich 2(DE)**

(54) **Three-dimensional scanner.**

(57) A three-dimensional scanner has at least three equi-distant circumferentially spaced beam scanning assemblies (8) between which are interposed sensing units (7), the assemblies and units being operatively in a fixed position relative to an object. The scanning assemblies (8) and sensing units (7) are mounted on a table which may be raised or lowered and arranged so that re-entrant profiles of the object which is approximately centrally disposed between the assemblies (8) and units (7) may be determined. Each scanning assembly receives light from a radiant energy source and scans the radiant energy around a portion of an object in discrete steps of, for example, one degree so that in total the entire 360° circumference of the object is illuminated by radiant energy. The sensing units detect and record profile information from the object at each illuminated step so that by using a triangulation calculation the true profile of the object may be deduced and a complete 360° representation of the object may be stored in memory. The profile information may be used in a medical environment where it is required to manufacture a corrective or supportive brace or when cosmetic restoration or enhancement is desired. The scanner is thus able to produce profile information of an object by using scanning assemblies and sensing units which are stationary with respect to an object.

Fig. 5

## THREE-DIMENSIONAL SCANNER

This invention relates to a three-dimensional scanner particularly, although not exclusively, for use in a medical environment.

In the fitment of prosthesis, wherever corrective or supportive braces are required or when cosmetic restoration or enhancement is contemplated, there is a requirement for information concerning human body profiles and currently such information is provided by plaster casts or mechanical measuring using vernier calipers. Such methods require skin contact which even on the slightest touch causes distortion. A non-contact method is therefore preferred and an apparatus for performing such non-contact measurement is disclosed in U.S.A. Patent No. 3,690,242. From U.S.A. Patent No. 3,690,242 it is known to provide a scanning apparatus for producing a three-dimensional sculpture from an object, such as a human head, but the known arrangement requires relative motion between the object and the scanning apparatus resulting in mechanical complexity and is unsuitable for medical use.

It is a principle object of this invention to provide a three-dimensional scanner which may be used in a medical environment.

According to this invention there is provided a three-dimensional scanner including:

(a) at least three scanning means mounted for peripheral location about an object to be scanned and at a fixed position in relation thereto, each said scanning means comprising radiant energy means, reflecting means for irradiating converging viewpoints of the object such that re-entrant portions of said object may be scanned, and means for 360° scanning the radiant energy around the object;

(b) at least three sensing means each adapted to detect radiant energy reflected from said object of said irradiation, each sensing means being associated with a respective scanning means and being mounted to detect reflected energy around 360° of the object; and

(c) data recording means arranged to sequentially receive information from the sensing means whereby 360° representation of a portion of said object is recorded.

Conveniently the scanning means includes means for progressing the radiant energy around the object in a stepwise fashion.

Preferably the data recording means is arranged to produce output signals to a profile shaping means for producing a required structure.

Advantageously the scanning means are arranged equi-distant on a framework around the object to be scanned.

Although each scanning means include an interruptable light source and optic means arranged to illuminate a narrow portion of the object to be scanned, or a single light source may supply light to all the scanning means, or in a currently preferred embodiment an interruptable light source is arranged to supply light to a pair of scanning means.

Preferably the scanning means comprises a line optic for producing the narrow light beam, a rotatable prism for receiving the narrow light beam and for applying said light beam to a light splitter arranged to apply light to reflecting means on opposing sides thereof, said reflecting means being adapted to direct light inwardly toward the object so that re-entrant portions thereof are illuminated, and means for rotating said prism in a stepwise fashion whereby said object is scanned by the narrow beam of light by rotation of said prism. Conveniently the rotatable prism and the light splitter are both line etched so that the light beam is rotatable in discrete steps, preferably each of a 1° angle.

Advantageously a shutter means is provided for limiting the total length of the line reflected by the opposed reflecting means.

Preferably each sensing means is located between two scanning means and each sensing means comprises a split reflective located between opposed gathering reflective members so that each narrow beam of light illuminating the object may be sensed, and memory means are provided for storing each sensed image.

Advantageously the memory means is one of a video camera, a video disc, and a Random Access Memory.

Preferably the data recording means is a central processor unit having a memory arranged to receive the information stored by each sensing means in turn so that the information determined by each of the sensing means is recorded thereon for utilisation.

Conveniently a radiant energy source is provided which is in the visible or invisible range, preferably a laser. Advantageously, to provide the interruption to the light source an interrupt disc is used to strobe the light source.

Advantageously the light source is arranged to direct light to a fibre optic, thence to a concentration lense and thence to the optic means.

Preferably datum means are provided of known spatial position with respect one another and to which sensed information of each sensing means may be correlated.

Conveniently the information stored in the central processor unit memory is arranged to be projected onto a display screen and advantageously means are provided for producing a magnification factor in the projection onto said display screen.

The scanner may be used with advantage to provide information concerning the change in dimension of an object with time and in such an embodiment preferably a sensed record of a profile is recorded on a recording means and arranged to be projected onto a display screen in combination with updated sensed profile information whereby the two sets of profile information are arranged to overlap on the display screen so that changes in the profile dimensions may be viewed or otherwise determined. In this respect it is envisaged that the display screen may be a virtual display screen with the changes in profile dimensions being electronically determined for subsequent utilisation. Additionally the updated profile information may be pre-recorded or projected in real-time.

As an example the use of the scanner will be considered in the case of amputations. In such cases, it is envisaged that the patient will be scanned a few days after the amputation, the time lapse being dictated by the time required to reduce the post-operative swelling, and any requirement to drain accumulating fluid around the wrap over flap. So as to provide an accurate artificial limb socket, the patient's stump is required to be scanned and the information on the profiles thus obtained will be viewed by a Clinician and a Technician, the Clinician deciding which areas of the scanned information he wishes to alter for pressure grip etc., and the Technician proceeding with the manufacture of the prosthesis. The scanner may also be used to make a record of a patient's good limb and the scanned profilometry is then reverse imaged to manufacture a flexible cover for the limb prosthesis. Such a proceedure could be applied to legs, feet, arms and hands etc.

Another proposed use for this invention relates to limb fractures. The present practice of using traction to reposition a break and using a plaster cast to immobilise the limb has a number of disadvantages. In this respect plaster bandages are messy and heavy, and while setting times have been reduced, there is still a time element for the plaster to dry. Once a cast is on a limb, weight is substantial requiring the patient to use considerable energy in movement and in the very young or elderly it is possible for the weight of the plaster to produce undesirable side effects such as, in a full foot to hip cast, severe muscular strain caused by body twist movement to throw the leg forward with the plaster cast. Also during the period in which the fracture is mending, it is the practice to cut the cast away once or twice for clinical examination and cast replacement is then required. With the scanner of the present invention it is possible to scan the broken limb while in traction then to directly cut a preformed limb shape block to produce a thermo set thin strong plastic case. Such a case may be set on a limb while the limb is still in traction and locked in position to the limb to provide support and immobilisation as in the plaster cast but such a plastic case would be light in weight and it would be possible to unlock the cast for investigation of the limb and to replace the case thereafter.

The invention will now be described by way of example with reference to the accompanying drawings in which:-

Figure 1(a) is a schematic front view illustrating the principle of operation employed in the calculations used for the present invention;

Figure 1(b) is an orthogonal view to that shown in 1(a);

Figure 2(a) is an embodiment illustrating the principle of operation used in the calculations employed in this invention;

Figure 2(b) is a view of part of the arrangement shown in Figure 2(a);

Figure 3 illustrates in schematic form the principle of illuminating an object in accordance with the invention;

Figure 4 shows a side view of the scanner in accordance with this invention;

Figure 5 shows a top view of the scanner shown in Figure 4;

Figure 6 shows raising and lowering the scanner;

Figure 7 shows an alternative arrangement for raising or lowering the scanner;

Figures 8(a) and 8(b) show mutually orthogonal views of a light scanning arragement used in the scanner of this invention;

Figure 9 shows a light sensing arrangement used in the scanner of this invention;

Figures 10(a) and 10(b) show mutually orthogonal views of an arrangement for utilising the sensed profile information; and

Figures 11(a) and 11(b) show mutually orthogonal views of an arrangement for comparing recorded images with one another.

Before describing the scanner of this invention the triangulation method of calculating the true dimensions of an object being scanned will be briefly described although detailed discussion of the single triangulation method is not thought necessary since the principle is already known and utilised in the art, for example in U.S.A. Patent No. 4,613,234.

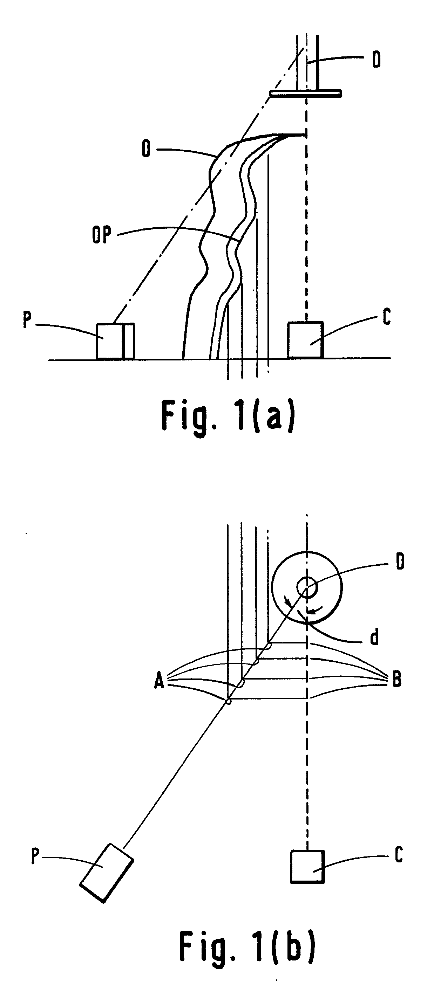

Referring to Figure 1(a) a single triangulation arrangement is shown where a central datum D is formed by a disc of known diameter and an object O is rotated about the datum. A camera C is located on the vertical sight line of the datum and a projector P is aligned on the datum D at 30° with respect to the camera, the parts being in fixed relationship to one another. Alternatively the object position is fixed and the camera and projector rotate about the object maintaining their fixed dispositions with respect one another and the object. As will be seen from 1(b) the camera C and projector P form a triangle with the datum D, the angle d being the apex angle with the distance between camera and projector forming the base. The projector P is arranged to throw a vertical split of light on to the object O so that a profile OP of the object is illuminated. In Figure 1(a) the view is directly along the camera sightline so that the datum D and the object profile OP are seen. The illuminated profiles are recorded in a regular angular fashion, for example in 1° steps over 360° with the recording being on any suitable storage medium such as photographic film or magnetic or semi-conductor storage. As seen from Figure 1(a) the datum and the camera sightline are in a common vertical plane and because the disc datum is a known height from the base of the object so it is possible to relate any point on the display profile to a dimension up or down on the vertical. Thus each point on the displayed profile can be given two dimensions, namely the angular rotation from the starting point of 0° and a height from the base. The third dimension, distance from datum D to the relevant point on the profile requires a triangulation calculation. The distance between a profile point referred back to the sightline B to the point on the object profile A as seen from the camera is a line parallel to the base line CP and the line BA is perpendicular to the sightline CD. A right angle triangle DBA is therefore formed and what is required to be calculated is the distance between the point A on the profile to the datum D. Knowing the angle d is 30° and the length BA, so DA may be calculated from $DA = BA \div \sin d$. The true lengths DA may therefore be rapidly calculated using a computer processor unit.

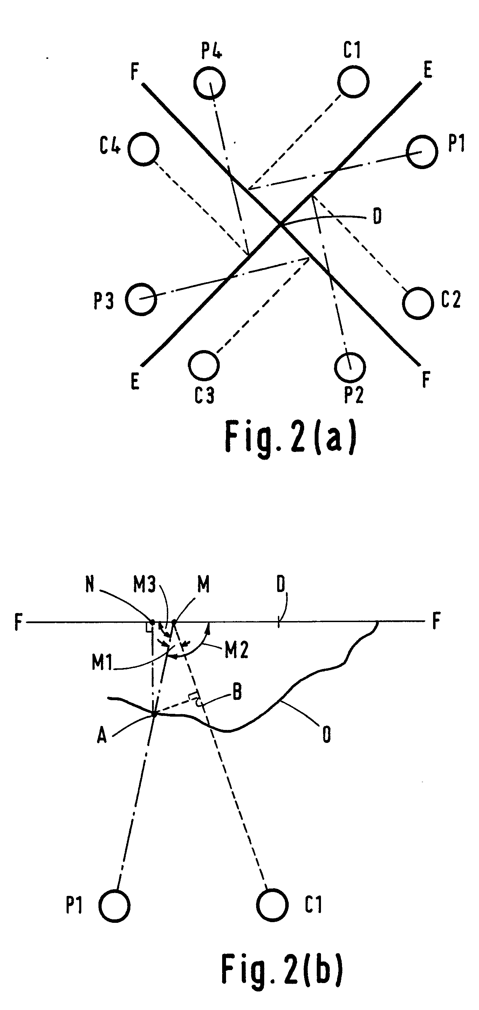

In multiple triangulation as shown in Figures 2-(a) and 2(b), although the basic principles of single triangulation as described above apply, there are differences which will now be discussed.

In multiple triangulation there is no rotational movement of the object or equipment and the only movement is of the projected beam. It therefore follows that the angles d will change as will the perceived position of the datum generally and so reference to a fixed datum is required. A datum plane is used for each set of light beam source and a camera equipment used with a minimum of three, but depending upon the object four or more sets are preferred to give better results. Referring to Figures 2(a) and 2(b) apparatus comprising a light beam source and a camera is required and means for traversing the beam about the object. Cameras C1, C2, C3, C4 each have a predetermined angular relationship with respective projector P1, P2, P3, P4, with hypothetical datums E-E and F-F being produced which intersect at a known central datum D that is used as a measurement point from which plus or minus measurements are taken. Camera C1 and projector P1 are on one side of plane F-F with the corresponding opposite pair of camera C3 and projector P3 being on the opposite side of the plane F-F. Similarly C2, P2 and C4, P4 act on opposing sides of plane E-E. Assuming for illustration purposes a single slit of light is projected at object O then the light beam strikes the object at profile point A and if continued meets the datum F-F at point M. The angular relationship between C1 and P1 is known so that angle M1 is known and the angle M2 from the sightline to the plane F-F is also known. The length MA may be calculated by the triangulation equation given above, i.e. $MA = BA \div \sin M1$, and since M2 is known, angle M3 can be calculated and the length NA which is a perpendicular from plane F-F to A may be calculated from $NA = MA \div \sin M3$. Thus the calculation may be made for each point on the profile and the distance each point N is referred back to the datum D.

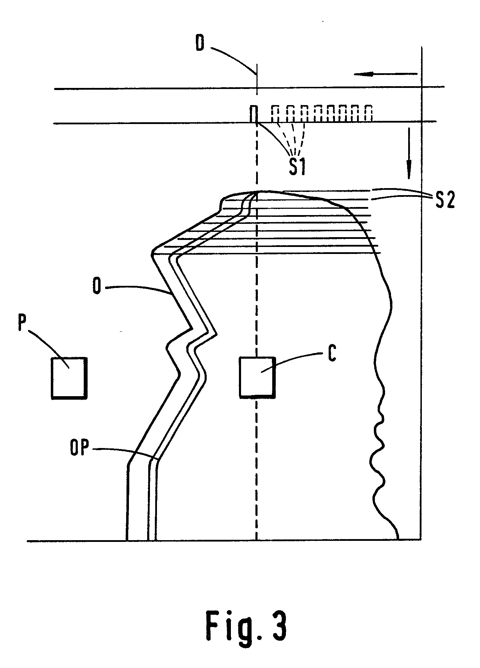

In the present invention an object is illuminated by light from a laser projector P as shown in Figure 3 and the beam is progressed around the object O in 0.50 in (1.27 mm) steps S1 (as shown by the horizontal arrow headed line) and dimensions are taken between the datum D and a point on the object in a vertical direction in 0.10 in (0.25 mm) steps S2 (as shown by the vertical arrow headed line).

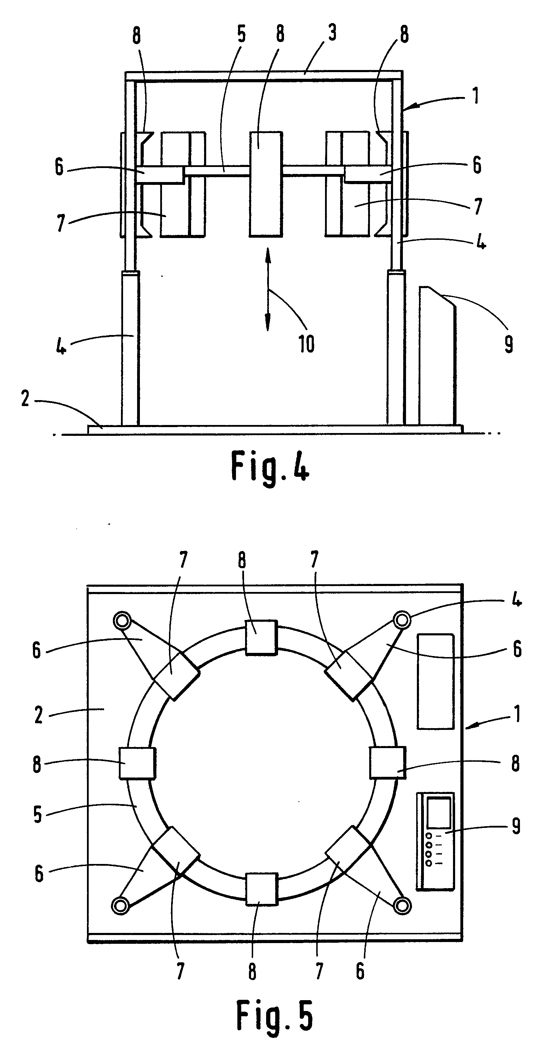

The scanner of this invention shown in Figures 4 and 5 has a frame 1 made of cast or toughened alloy comprising a base plinth 2 and a top square frame 3 support by four pillars 4 which are height adjustable as shown by the double arrow headed line 10. A circular member 5 is secured by equi-circumferentially spaced arms 6 to the movable parts of the pillars 4 and located on each of the four arms 6 is a sensing unit 7. A scanning assembly 8 is located intermediate each of the sensor assemblies. A control panel 9 is located to one side of the base plinth 2.

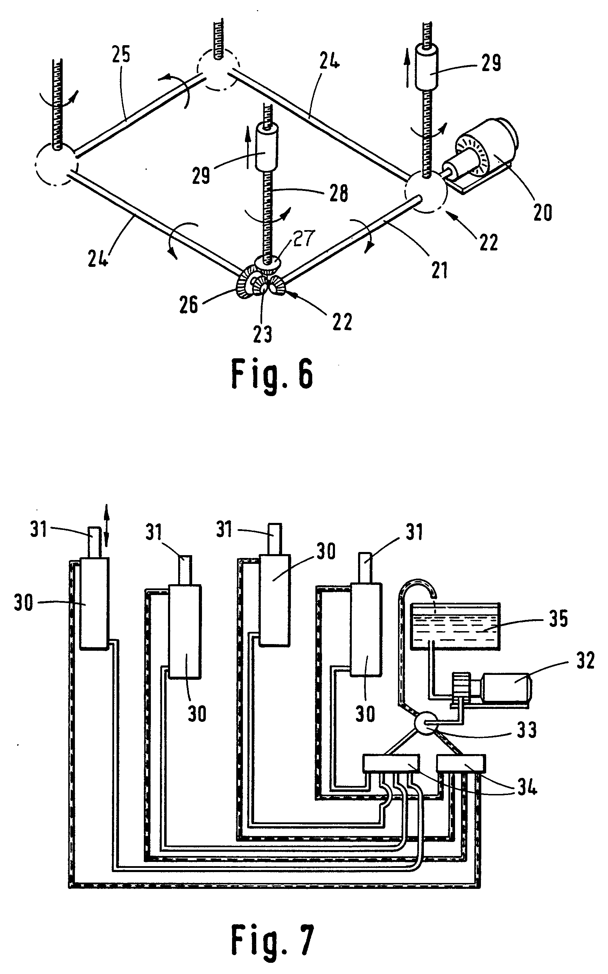

Referring to Figure 6 there is shown an electro-mechanical apparatus for raising the pillars 4. In Figure 6 a motor 20 with variable speed/drive is located to drive a first shaft 21 having a bevel gear 22 at opposing ends thereof. Each of the bevel gears 22 drives an associated bevel gear 23 of a slave shaft 24. The slave shaft 24 also has at its end opposite to its connection with shaft 21 further bevel gears for rotation of another slave shaft 25. Located on each of the shafts 21, 24, 25 is a gear wheel 26 arranged for driving a pinion 27 fixedly connected to a crew thread column 28 upon which a non-rotable meshingly connected collar 29 is located. The collar 29 is connected to the arms 6. In operation, with rotation of the motor 20 so the collar 29 is driven up or down in dependence upon the direction of rotation of the motor to thereby raise or lower the circular member 5.

In an alternative arrangement shown in Figure 7 for raising and lowering the circular member 5, four hydraulic rams 30 having pistons 31 for attachment to the arms 6 are driven over interconnecting lines from a pump 32 via a control valve 33 and balance valves 34. Hydraulic coil for the apparatus of Figure 4 is stored in a reservoir 35.

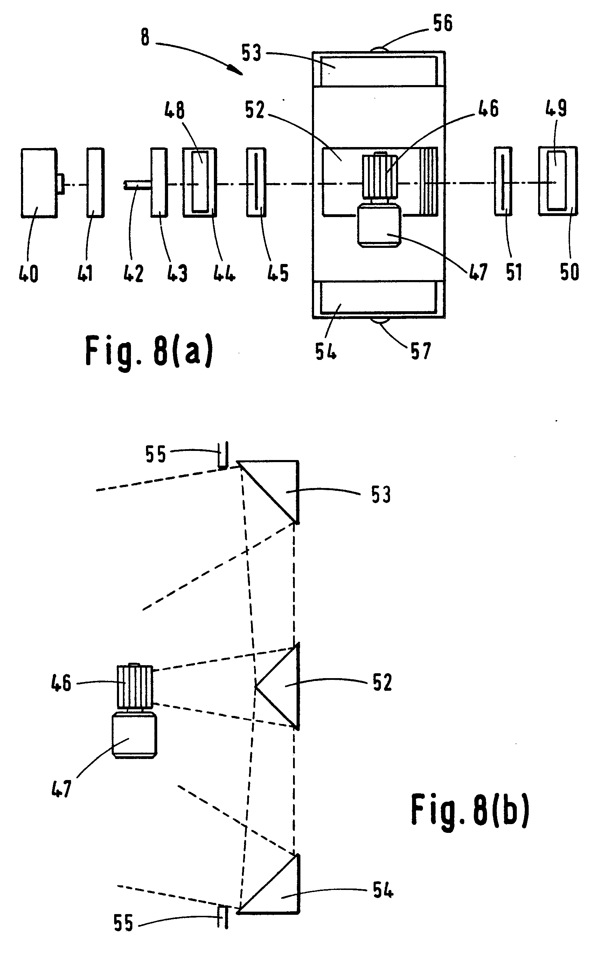

One of the equi-distant spaced beam scanning assembly 8 is shown in Figures 8(a) and 8(b) and has a laser 40 projecting a light beam through a shutter 41 thence over a fibre optic cable 42 to a concentration lens assembly 43. The concentrated light passes through a semi-silvered mirror 44 to a line optic 45 so that a narrow line of light is formed which is transmitted to one side of a rotatable, line etched prism 46 driven in a stepwise fashion by a stepping motor 47. The mirror 44 transmits half the applied light to the line optic 45 and the other half if applied via reflectives 48, 49 and 50 to another line optic 51 which applies a narrow line of light to another side of the prism 46. The prism 45 thus transmits a pair of light beams to each side of a double angled, line etched light splitter 52 so that the light is transmitted into two opposing paths which in the present example are an upper path and a lower path. The light in the two paths is transmitted to an upper reflective 53 and a lower reflective 54 respectively which each then transmit the narrow light beam toward the centre of the circular member 5 in which an object being scanned is, in operation, positioned so that the light beams from the upper and lower reflectives converge upon the object. The effect obtained is of one long single reflected line but sourced from above and below the subject to be scanned and the line of light is progressively swept across reflectives 53, 54 and thereby stepped across the object in a predetermined fashion of approximate 0.050 inch (1.27 mm) steps by the rotation of prism 46. Discrete steps are ensured by the use of line etched members 46 and 52. Sliding shutters 55 are used to limit the total length of the reflected line. Located above and below each scanning assembly are single fibre optic cables 56, 57 which face toward the subject so as to provide a known fixed datum point as a reference for the sensor units other than the sensor unit sensing the lines produced by the beam securing assembly with which they are associated. Examples of the light paths are shown in broken lines in Figure 8(b).

By arranging the upper and lower reflectives 53, 54 to provide a converging light beam above and below the centre line of the object respectively re-entry profiles may be scanned.

In a currently preferred embodiment the laser 40 is arranged to feed two scanning assemblies via a beam splitter so that two lasers are required in the embodiment described herein each feeding a pair of adjacent scanning assemblies. Although it is envisaged that one laser could provide the required light output for all of the scanning assemblies it has been found that such an arrangement is currently unsatisfactory due to light attenuation in the required fibre optic couplers.

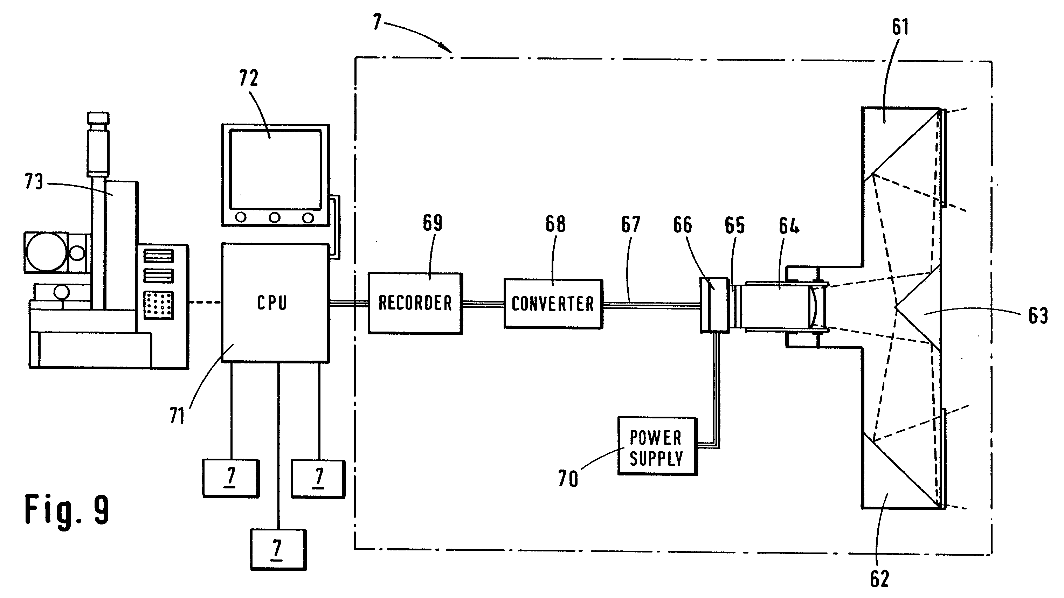

A sensing unit 7 is shown in Figure 9 and one such unit is associated with each of the scanning assemblies. Each sensing unit has an upper gathering reflective 61 and a lower gathering reflective 62 which are arranged to receive reflected light energy from the object being scanned. The upper and lower reflectives reflect received light to a fixed split reflective 63 which in turn reflects the received light to a lens assembly 64. Having been appropriately focused the received light image is focused onto a picture element matrix display semi-conductor chip 65 if the type currently used in, for example, a video camera; a RAM may alternatively be used. Signals, representative of the grey scale, from each of the elements of the display 65 are read off and transmitted in a known manner by an electro optic circuit 66 in sequential form over a fibre optic cable 67 to an opto-electric signal converter 68. A fibre optic cable 67 is used primarily due to its lower susceptibility to noise and for its greater signal handling capabilities. The signal converter 68 applies the signals in suitable form to a video cassette recorder 69. The display 65, circuit 66, signal converter 68, video cassette recorder 69 may be elements from a known video camera/recorder combination in which signals on the semi-conductor chip pixels are transmitted to the recorder. It is also envisaged that instead of a video cassette recorder, a video disc or a random access memory (RAM) may be used.

Thus each recorder 69 receives a progressive line scan for each quadrant that is illuminated.

Each frame of scanned information carries a view of the object profile illuminated by the narrow light beam in 0.010 inches (0.25 mm) steps from the datum to the beam edge with the fibre optic cables 56, 57 of two of the opposing scanning assemblies being illuminated so that four fixed datums are provided which remain constant on each frame irrespective of how the line illumination is moved around the object. The fact that these datums are fixed, so dimensional and angular profile information may be related to the datums such that error may be eliminated in subsequent utilisation of the scanned information.

**EP 0 305 107 A2**

A power transformer and stabilised source 70 is used for a power source to the circuit 66.

Each of the four recorders 69 in each sensing unit 7 is connected to data central processing unit (CPU) 71 which may include a tape or video disk memory in such a manner that the information concerning each of the four quadrants stored by the respective recorders 69 is extracted in sequence either clockwise or anti-clockwise and stored on the memory. In this respect the complete information from each recorder 69 is transferred in sequence to the CPU memory. Upon completion of data acquisition on the memory, the CPU uses the triangulation calculations to give the true profile which is then stored. An operator may replay the data in a predetermined slow speed and view it on a digitising monitor 72 so that the information stored on the CPU 71 is a 360° representation of the part of the object that was scanned.

The digitising monitor 72 is provided to digitise the 360° information held by the CPU 71 frame by frame and to subsequently pass the digitised information to a numerical cutting machine 73 for cutting the required prosthesis profile or other required cast. Alternatively, each frame may be projected and a profile cut as described in USA Patent No. 3,796,129. In another alternative the CPU 71 may provide an output signal representative of each pixel signal amplitude to a printer.

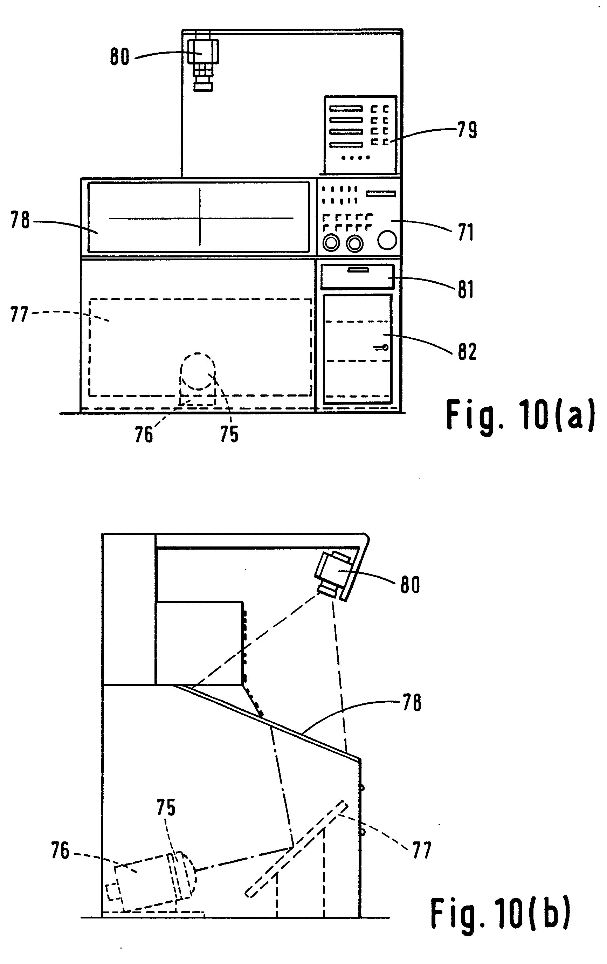

Prior to digitising, it is possible for a Clinician to alter profiles to provide desired pressure points in the prosthesis or cast to be cut and one such digitising assembly is shown in Figures 10(a) and 10(b).

The digitising assembly shown in Figures 10(a) and 10(b) display the frame by frame information stored by the CPU 71 memory via a cathode ray tube 76 and magnifying projection lens assembly 75 onto a reflective member 77 angled at 30° to the vertical and thence onto a screen 78. The projected datums which originated from the datums 50, 51 are checked for correct location either using a measuring rule directly or alternatively using a Heidenham measuring system known per se in conjunction with digital read-outs on display panel 79. In the Heidenham system, either linear gratings and magnification optics or optical encoders are used for measuring purposes. The linear gratings are read directly by a cursor and magnified but if an optical encoder is used a rotating linear grating and light pulses caused by the bars of the grating provide signals to operate the digital read-out. If there is any error in the positioning of the datums this is corrected by adjustment of the projection elements. The total of eight pairs of datum points are registered on the screen 78 and each following frame is required to have its datum points aligned on the registers to ensure correct data acquisition.

A Clinician has an overlay at the magnification scale of the projection apparatus which is an overlay of acetate for blanking out an undesired section of the projected profile or to insert a modified profile section as required. A digitising camera 80 located above the screen 1 is operated and the information on the screen collected and digitised. The angular position is entered in a computer 81 having memory storage 82 together with the factor of magnification of the projection on the screen 78 and when all the profile data has been entered into the computer storage then the computer is able to reduce the dimensions to actual scale and apply the trigometrical factor for angular correction as will be well appreciated by the person of ordinary skill in the art. Either a punched or magnetic tape is provided therefrom for machine instructions in multi-axis dimensioning for use by the numerical cutting machine 73.

For saving of costs it is currently envisaged that the information gathered will be in monochrome since the cost of adapting the apparatus for colour is not currently thought worth while.

There are circumstances in medical and other applications where it is required that the profile geometry of one or more sections of a person's profile or object be compared over a period of time. A typical example might be to assess the rapidity of growth of a tumour just under the skin in the spinal or other area.

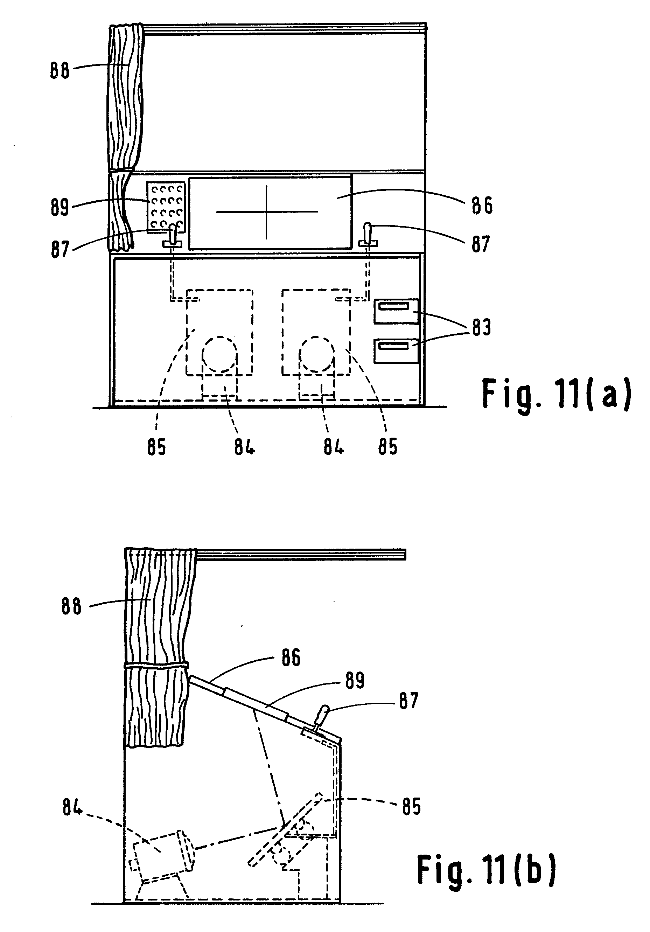

The apparatus shown in Figures 4 to 9 would be used to record full profile information on the memory of CPU 71, which, as previously stated, could be video tope or disk. At a predetermined later period of time the subject is again placed in the scanner assembly in exactly the same position as previously and a second scan of the area which profile is taken is again recorded. The original scanning record is then compared with the updated scanning record utilising the equipment shown in Figures 11(a) and 11(b).

The equipment shown in Figures 11(a) and 11-(b) comprises a pair of cathode ray tube projectors 84 similar to the CRT 76 and lens assembly 75 of Figures 10(a0 and 10(b). The projectors 84 are directed toward a reflective 85 which throws an image onto a display screen 86. A pair of video tape or video disk replay units 83 are provided for receiving the information recorded by CPU 71 at the original and updated scan and the CRT projectors 84 are each arranged to project a different coloured image which may include white. Each of the reflectives 85 is adjustable by an associated joy stick 87 to ensure that reflected images from each of the projectors 84 are aligned with one another using the datum fibre optic cables 56, 57 upon the display screen 86. A control panel 89 is provided for the replay units 83 and the projectors 84 and it is envisaged that the projectors 84 may be fed directly from CPU memory 71 if desired by ensuring that the CPU memory 71 is provided with the original and updated scanned information. A curtain 88 is provided which can be drawn around the screen 86 to enable better sight of the screen.

In use of the apparatus shown in Figures 11(a) and 11(b) signals from each of the video replay units 83 are fed to CRT projectors 84 and the images projected by the CRT 84 are arranged to be of differing colours for each of the recorded images by any known means of providing a coloured image which may include the use of filters. Thus it is envisaged, for example, that the original profile recording may be projected in a red colour and the updated profile display recording may be projected as a white image. The images from the projectors 84 are adjusted to align with one another by motion of the reflectives 85 using joy sticks 87 by the operator ensuring that the four datums from each scanning exactly overlie one another. An examination of the displayed profile would show only the dominant colour, say red, if no increase in growth has occured. If growth has occured then there will be an area where the profile from the updated scanning record will not exactly overlie the original scanning record and white may be visable. Similarly if radiation treatment or some other treatment has been used on a tumour causing a reduction in the size of the tumour by internal cell destruction then again the image reduction will be visable. The CRT projectors 84 with the reflectives 85 and screen 86 may be arranged to provide a magnification of 10:1 or any other magnification as required. Thus a Clinician may physically measure on the screen 86 differences between the two scanning records by allowing for the magnification factor and accurately state an increase or decrease.

In an alternative method a person or object may be retained in the scanner of Figures 4, 5 and a direct feed made from the sensor 7 and scanner 8 to one of the projectors 84 while the previous sensed record is replayed by one of the replay units 83 to the other of the CRT projectors 84. Thus by a strip by strip review of the two images the change in dimension of the profile may be determined.

It will also be realised that although four scanning and sensing assemblies have been described, if the assemblies are moved sufficiently far away from the object to be scanned then only three scanning and sensing assemblies are required, the essential requirement of course being that the whole 360° of the object is illuminated and sensed. Moreover, other optical arrangements will be evident to those skilled in the art, the fundamental requirement being that 360° scanning is effected.

## Claims

1. A three-dimensional scanner including:

(a) at least three scanning means (8) mounted for peripheral location about an object to be scanned and at a fixed position in relation thereto, each said scanning means (8) comprising radiant energy means (40), reflecting means (52-54) for irradiating converging viewpoints of the object such that re-entrant portions of said object may be scanned, and means (46, 47) for 360° scanning the radiant energy around the object;

(b) at least three sensing means (7) each adapted to detect radiant energy reflected from said object of said irradiation, each sensing means being associated with a respective scanning means (8) and being mounted to detect reflected energy around 360° of the object; and

(c) data recording means (71) arranged to sequentially receive information from the sensing means (7) whereby 360° representation of a portion of said object is recorded.

2. A scanner as claimed in claim 1 wherein the scanning means (8) includes means (46) for progressing the radiant energy around the object in a stepwise fashion.

3. A scanner as claimed in claim 1 or 2 wherein the data recording means (71) is arranged to produce output signals to a profile shaping means for producing a required structure.

4. A scanner as claimed in any preceding claim wherein the scanning means (8) are arranged equi-distant on a framework (1-5) around the object to be scanned.

5. A scanner as claimed in claim 4 wherein each scanning means (8) include an interruptable light source (40, 41) and optic means (43-45; 48-51) arranged to illuminate a narrow portion of the object to be scanned.

6. A scanner as claimed in claim 4 including a single light source (40) for supplying light to all the scanning means (8).

7. A scanner as claimed in claim 4 including an interruptable light source arranged to supply light to a pair of scanning means.

8. A scanner as claimed in any preceding claim wherein the scanning means (8) comprises a line optic (45) for producing the narrow light beam, a rotatable prism (46) for receiving the narrow light beam and for applying said light beam to a light splitter (52) arranged to apply light to reflecting means (53, 54) on opposing sides thereof, said reflecting means (53, 54) being adapted to direct light inwardly toward the object so that re-entrant portions thereof are illuminated, and means (47) for rotating said prism in a stepwise fashion whereby said object is scanned by the narrow beam of light by rotation of said prism.

9. A scanner as claimed in claim 8 wherein the rotatable prism (46) and the light splitter (52) are both line etched so that the light beam is rotatable in discrete steps.

10. A scanner as claimed in claim 9 wherein each step is a 1° angle.

11. A scanner as claimed in claim 8 wherein a shutter means (55) is provided for limiting the total length of the line reflected by the opposed reflecting means (53).

12. A scanner as claimed in any preceding claim wherein each sensing means (7) is located between two scanning means (8) and each sensing means (7) comprises a split reflective (63) located between opposed gathering reflective members (61, 62) so that each narrow beam of light illuminating the object may be sensed, and memory means (69) are provided for storing each sensed image.

13. A scanner as claimed in claim 12 wherein the memory means (69) is one of a video camera, a video disc, and a Random Access Memory.

14. A scanner as claimed in any preceding claim wherein the data recording means (71) is a central processor unit having a memory arranged to receive the information stored by each sensing means (7) in turn so that the information determined by each of the sensing means is recorded thereon for utilisation.

15. A scanner as claimed in claim 1 wherein the wavelength of the radiant energy means is in the visible range.

16. A scanner as claimed in claim 1 wherein the wavelength of the radiant energy means is in the invisible range.

17. A scanner as claimed in claim 15 wherein the radiant energy means is a laser (40).

18. A scanner as claimed in claim 5 wherein the interruptable light source includes an interrupt disc (41) for strobing the light source.

19. A scanner as claimed in claim 18 wherein the light source (40) is arranged to direct light to a fibre optic (42), thence to a concentration lense (43) and thence to the optic means (43-45; 48-51).

20. A scanner as claimed in any preceding claim wherein datum means (56, 57) are provided of known spatial position with respect one another and to which sensed information of each sensing means may be correlated.

21. A scanner as claimed in claim 14 wherein the information stored in the central processor unit (71) memory is arranged to be projected onto a display screen.

22. A scanner as claimed in claim 21 wherein means (75-78) are provided for producing a magnification factor in the projection onto said display screen.

23. A scanner as claimed in claim 1 wherein a sensed record of a profile is recorded on a recording means (83) and arranged to be projected onto a display screen (86) in combination with updated sensed profile information whereby the two sets of profile information are arranged to overlap on the display screen so that changes in the profile dimensions may be viewed or otherwise determined.

Fig. 1(a)

Fig. 1(b)

Fig. 2(a)

Fig. 2(b)

D

S1

S2

O

P

C

OP

Fig. 3

Fig.4

Fig.5

Fig. 6

Fig. 7

Fig. 8(a)

Fig. 8(b)

Fig. 9

72

73

CPU

71

7    7

7

7

69 RECORDER

68 CONVERTER

67

66

65

64

70 POWER SUPPLY

61

63

62

EP 0 305 107 A2

Fig. 10(a)

Fig. 10(b)

Fig. 11(a)

Fig. 11(b)